# DEMANDE DE BREVET EUROPEEN

(11) **EP 1 317 919 A1**
(43) Date de publication de la demande: **11.06.2003**
(21) Numéro de dépôt: 02292841.0
(22) Date de dépôt: 14.11.2002
(51) Int. Cl.: A61K 7/42

(54) **Compositions cosmétiques antisolaires à base d'un mélange synergique de filtres et utilisations**

(30) Priorité: 07.12.2001 FR 0115861
(71) Demandeur: l'Oreal SA, 75008 Paris (FR)
(72) Inventeur: Candau, Didier, 91570 Bievres (FR)
(74) Mandataire: Miszputen, Laurent

(57) **Abrégé**

L'invention concerne de nouvelles cosmétique ou dermatologique à usage topique, caractérisée par le fait qu'elle comprend, dans un support cosmétiquement acceptable, au moins :
(a) 0,1 à 15% en poids par rapport au poids total de la composition d'acide benzène 1,4-di(3-méthylidène-10-camphosulfonique), éventuellement sous forme partiellement ou totalement neutralisée, à titre de premier filtre et
(b) 0,1 à 15% en poids par rapport au poids total de la composition d'au moins un dérivé de 2-hydroxybenzophénone amino-substitué particulier ; lesdits premier et second filtres étant présents dans lesdites compositions dans une proportion produisant une activité synergique au niveau des facteurs de protection solaires conférés.

L'invention concerne également leurs applications à la protection de la peau et des cheveux contre les effets du rayonnement ultraviolet.

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins deux filtres particuliers, à savoir d'une part l'acide benzène 1,4-di(3-méthylidène-10-camphosulfonique) et, d'autre part, un composé 2-hydroxybenzophénone amino-substitué particulier. L'association de ces deux filtres conduit à l'obtention d'un effet de synergie au niveau des indices de protection conférés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber selectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV.

On connaît dans la demande de brevet EP1046391 des compositions solaires à base de dérivés de 2-hydroxy benzophénone aminosubstitués. Ces composés filtrent les radiations UV-A.

Or, à la suite d'importantes recherches menées dans le domaine de la photoprotection évoqué ci-dessus, la Demanderesse a découvert, de façon inattendue et surprenante, que la combinaison de deux filtres solaires particuliers et déjà connus en soi dans l'état de l'art, permettait, du fait d'un effet de synergie remarquable, d'obtenir des compositions antisolaires présentant des indices de protection nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques ou dermatologiques à usagé topique, caractérisées par le fait qu'elle comprennent, dans un support cosmétiquement acceptable, au moins :
(a) 0,1 à 15% en poids par rapport au poids total de la composition d'acide benzène 1,4-di(3-méthylidène-10-camphosulfonique), éventuellement sous forme partiellement ou totalement neutralisée, à titre de premier filtre et
(b) 0,1 à 15% en poids par rapport au poids total de la composition d'au moins un dérivé de 2-hydroxybenzophénone amino-substitué de formule (I) que l'on définira ci-dessous , à titre de second filtre ; lesdits premier et second filtres sont présents dans les compositions selon l'invention dans une proportion produisant une activité synergique au niveau des facteurs de protection solaires conférés.

La présente invention a également pour objet l'utilisation de telles compositions pour la fabrication de compositions cosmétiques destinées à la protection de la peau et/ou des cheveux contre le rayonnement ultraviolet, en particulier le rayonnement solaire.

La présente invention a également pour objet l'utilisation d'un dérivé de 2-hydroxybenzophénone aminosubstiuté de formule (I) pour la fabrication de compositions cosmétiques ou dermatologiques destinées à la protection de la peau et/ou des cheveux contre le rayonnement ultraviolet, en particulier le rayonnement solaire comprenant au moins l"acide benzène 1,4-di(3-méthylidène-10-camphosulfonique), éventuellement sous forme partiellement ou totalement neutralisée, dans le but de produire un effet synergique au niveau des indices de protection solaire conférés.

D'autres caractéristiques, aspects et avantages de la présente invention apparaitront à la lecture de la description détaillée qui va suivre.

L'acide benzène 1,4-di(3-méthylidène-10-camphosulfonique) et ses différents sels décrits notamment dans les demandes de brevets FR-A- 2 528 420 et FR-A- 2 639 347, sont des filtres déja connus en soi (filtres dits à large bande) capables en effet d'absorber les rayons ultraviolets de longueur d'ondes comprises entre 280 et 400 nm, avec des maxima d'absorption compris entre 320 et 400 nm, en particulier aux alentours de 345 nm. Ces filtres répondent à la formule générale (II) suivante : 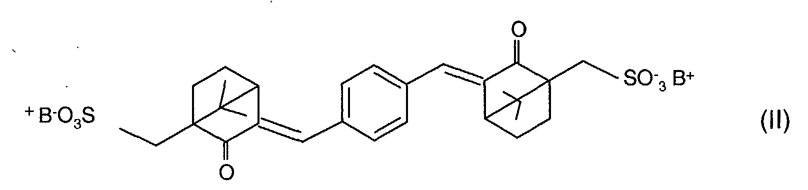 dans laquelle B désigne un atome d'hydrogène, un métal alcalin ou encore un radical NH(R)₃+ dans lequel les radicaux R, qui peuvent être identiques ou différents, désignent un atome d'hydrogène, un radical alkyle ou hydroxyalkyle en C₁-C₄ ou encore un groupement Mn+/p, Mn+ désignant un cation métallique polyvalent dans lequel p est est égal à 2 ou 3 ou 4, Mn+ désignant de préférence un cation métallique choisi parmi Ca²+, Zn²+, Mg²+, Ba²+, Al³+ et Zr⁴+. II est bien entendu que les composés de formule (I) ci-dessus peuvent donner lieu à l'isomère "cis-trans" autour d'une ou plusieurs double(s) liaison(s) et que tous les isomères rentrent dans le cadre de la présente invention.

L'acide benzène 1,4-di(3-méthylidène-10-camphosulfonique) et ses différents sels sont présents de préférence dans la composition de l'invention dans des proportions allant de 1 % à 10 % en poids, et plus particulièrement de 2 à 8% en poids par rapport au poids total de la composition.

Les dérivés de 2-hydroxybenzophénone aminosubstitués conformes à l'invention répondent à la formule (I) suivante : 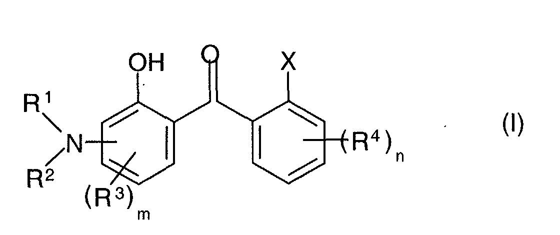 dans laquelle :
R¹ et R², identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C₁-C₁₂, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷;
R⁵, R⁶ et R⁷, identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀, un groupe -(YO)ₒ-Z ou un groupe aryle ;
Y désigne -(CH₂)₂-, -(CH₂)₃- -(CH₂)₄-, -CH-CH₃-CH₂- ;
Z représente -CH₂-CH₃, -CH₂CH₂CH₃, -CH₂-CH₂-CH₂-CH₃, -CH(CH₃)-CH₃ ;
m est un entier variant de 0 à3 ;
n est un entier variant de 0 à 3 ;
o est un entier variant de 1 à 2.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en C₂-C₁₀, on peut citer par exemple : vinyle, n-propènyle, isopropènyle, 1-butènyle, 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en C₁-C₁₂, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclopropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino ; C₁-C₄alkyle ; C₁-C₄-alcoxy ; hydroxy ; ils peuvent également comporter de 1 à 3 hétéroatomes comme souffre, oxygène ou azote dont les valences libres puvent être occupées par un hydrogène ou un radical alkyle en C₁-C₄.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; C₁-C₄-alkylamino ; C₁-C₄ dialkylamino; C₁-C₄alkyle ; C₁-C₄-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux R¹ et R² avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

Une famille de composés de formule (I) préférentiels comprend ceux choisis parmi ceux de formule (la) suivante : 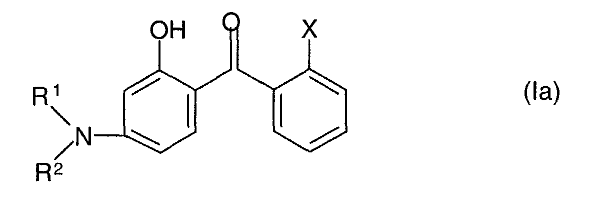 dans laquelle :
R¹ et R², identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷;
R⁵ désigne un atome d'hydrogène, un radical alkyle en C₁-C₁₂, un radical cycloalkyle en C₃-C₆.
R⁶ et R⁷, identiques ou différents, désignent un atome d'hydrogène, un radical alkyle en C₁-C₁₂, un radical cycloalkyle en C₅-C₆.

Les composés de formule (la) plus particulièrement préférés sont ceux pour lesquels :
R¹ et R², identiques ou différents, désignent un radical alkyle en C₁-C₄ et plus particulèrement éthyle ;
R⁵ désigne un radical alkyle en C₃-C₈,
R⁶ et R⁷, identiques ou différents, désignent un radical alkyle en C₁-C₈,
Une autre famille de composés de formule (I) préférentiels comprend ceux choisis parmi ceux de formule (Ib) suivante : 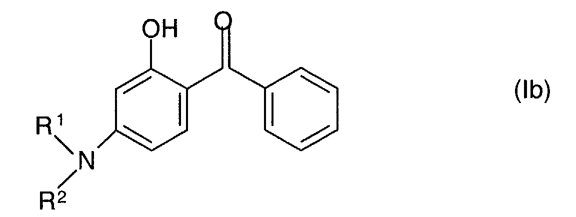
dans laquelle :
R¹ et R², identiques ou différents, désignent un radical alkyle en C₁-C₁₂ ou forment avec l'atome d'azote avec lequel ils sont liés un cycle à 5 ou 6 chaînons.

Parmi les composés de formule (Ib), on peut citer plus particulièrement :
- le (4-diéthylamino-2-hydroxyphényl)-phénylcétone.
- le (4-pyrrolidino-2-hydroxyphényl)-phénylcétone.

Une famille de composés de formule (I) plus particulièrement préférés comprend ceux choisis parmi ceux de formule (lc) suivante : 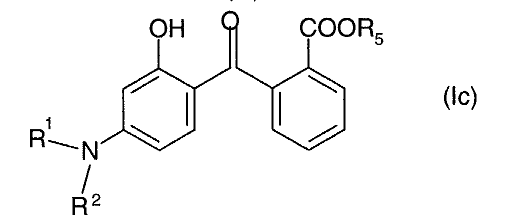 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

Parmi les composés de formule (lc), on peut citer :
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Un composé de formule (I) tout particulièrement préféré est le 2-(4-diéthylamino-2-hydroxybenzoyl)-benzoate de n-hexyle.

Les composés de formule (I) tels que définis ci-dessus sont connus en eux-mêmes et leurs structures et leurs synthèses sont décrites dans les demandes de brevet EP-A-1046391 et DE100 12 408 (faisant partie intégrante du contenu de la description).

Les dérivés de 2-hydroxybenzophénone aminosubstitués conformes à l'invention sont présents de préférence dans la composition de l'invention dans des proportions allant plus préférentiellement de 1 à 10% en poids et plus particulièrement de 2 à 8% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés cinnamiques ; les dérivés salicyliques, les dérivés du camphre autres que le composé (II) ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (I); les dérivés de β,β'-diphénylacrylate ; les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649; les 4,4-diarylbutadiènes tels que décrits dans les demandes EP0967200, DE19746654, DE19755649, EP-A-1008586.

Comme exemples de filtres organiques complémentaires actifs dans l'UV-A et/ou l'UV-B, on peut citer désignés ci-dessus sous leur nom INCI :

### Dérivés de l'acide Dara-aminobenzoique :

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

### Dérivés salicyliques:

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

### Dérivés du dibenzoylméthane :

- Butyl Methoxydibenzoylmethane vendu notamment sous le nom commercial « PARSOL 1789 » par HOFFMANN LA ROCHE,
- Isopropyl Dibenzoylmethane,

### Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- Diisopropyl Methylcinnamate,
- Glyceryl Ethylhexanoate Dimethoxycinnamate

### Dérivés de β,β'-diphénvlacrylate :

- Octocrylene vendu notamment sous le nom commercial « UVINUL N539 » par BASF,
- Etocrylene, vendu notamment sous le nom commercial « UVINUL N35 » par BASF,

### Dérivés de la benzophénone:

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

### Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Polyacrylamidomethyl Benzylidene Camphor fabriqué sous le nom « MEXORYL SW » par CHIMEX,

### Dérivés du phenyl benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

### Dérivés de la triazine :

- Anisotriazine vendu sous le nom commercial «TINOSORB S » par CIBA SPECIALTY CHEMICALS
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

### Dérivés du phenyl benzotriazole :

- Drometrizole Trisiloxane vendu sous le nom « SILATRIZOLE » par RHODIA CHIMIE ,
- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques:

- Menthyl anthranilate vendu sous le nom commercial commercial « NEO HELIOPAN MA » par HAARMANN et REIMER,

### Dérivés d'imidazolines :

- Ethylhexyl Dimethoxybenzylidene Dioxoimidazoline Propionate,

### Dérivés de benzalmalonate :

- Polyorganosiloxane à fonction benzalmalonate vendu sous la dénomination commerciale « PARSOL SLX » par HOFFMANN LA ROCHE et leurs mélanges.

### Dérivés de 4,4-diarylbutadiene

1,1-dicarboxy (2,2'-diméthyl-propyl)-4,4-diphénylbutadiène.
et leurs mélanges.

Les filtres UV organiques solubles plus particulièrement préférés sont choisis parmi les composés suivants :
- Ethylhexyl Salicylate,
- Octocrylene,
- Ethylhexyl Methoxycinnamate
- Butyl Methoxydibenzoylmethane,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Drometrizole Trisiloxane,
- Methylène bis-Benzotriazolyl Tetramethylbutylphénol
- 1,1-dicarboxy (2,2'-diméthyl-propyl)-4,4-diphénylbutadiène
et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les. demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprlque/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets, mais nullement limitatifs, illustrant l'invention, vont maintenant être donnés.

**Exemple 1**

| | |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en C₁₂-C₁₅ (WITCONOL TN -WITCO) | 10 g |
| 2-(4-diethylamino-2-hydroxybenzoyl)-benzoic acid hexylester | 2g |
| Glycérine | 10g |
| Terephtalylidene dicamphor sulfonique acid (MEXORYL SX - CHIMEX) | 2g |
| Triéthanolamine | qs pH7 |
| Conservateurs | qs |
| Eau déminéralisée qsp | 100 g |

| **Exemple 2** | |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C12/C15 (WITCONOL TN -WITCO) | 15g |
| Triéthanolamine | 0.5g |
| 2-(4-diethylamino-2-hydroxybenzoyl)-benzoic acid hexyleste | 2.5g |
| Glycérine | 5g |
| Phosphate d'alcool hexadécylique,sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| Terephtalylidene dicamphor sulfonique acid (MEXORYL SX - CHIMEX) | 1.5g |
| Triethanolamine | qs pH 7 |
| Conservateurs | qs |
| Eau démineralisée qsp | 100 g |

## Revendications

1. Composition cosmétique ou dermatologique à usage topique, **caractérisée par le fait qu'**elle comprend, dans un support cosmétiquement acceptable, au moins :
(a) 0,1 à 15% en poids par rapport au poids total de la composition d'acide benzène 1,4-di(3-méthylidène-10-camphosulfonique), éventuellement sous forme partiellement ou totalement neutralisée, à titre de premier filtre et
(b) 0,1 à 15% en poids par rapport au poids total de la composition d'au moins un dérivé de 2-hydroxybenzophénone amino-substitué de formule (I) suivante : 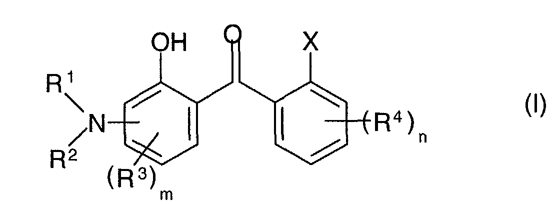
dans laquelle:
R¹ et R², identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀;
R¹ et R² peuvent également former avec l'atome d'azote avec lequel ils sont liés un cycle à 5 ou 6 chaînons ;
R³ et R⁴, identiques ou différents, désignent un radical alkyle en C₁-C₂₀, un radical alcènyle en C₂-C₁₀, un radical cycloalkyle en C₃-C₁₀, un radical cycloalcènyle en C₃-C₁₀, un radical alcoxy en C1-C12, un radical (C₁-C₂₀)alcoxycarbonyle, un radical alkylamino en C₁-C₁₂, un radical dialkylamino en C₁-C₁₂, un radical aryle ou un hétéroaryle éventuellement substitué, un substituant hydrosolubilisant choisi parmi un groupe carboxylate, un groupe sulfonate ou un reste ammonium ;
X désigne un atome d'hydrogène, un groupe COOR⁵ ou CONR⁶R⁷;
R⁵, R⁶ et R⁷, identiques ou différents, désignent un atome d'hydrogène, un radical alkyle en C₁-C₂₀, un radical alcènyle en C₂-C₁₀, un radical cycloalkyle en C₃-C₁₀, un radical cycloalcènyle en C₃-C₁₀, un groupe -(YO)ₒ-Z ou un groupe aryle ;
Y désigne -(CH₂)₂-, -(CH₂)₃- -(CH₂)₄-, -CH-CH₃-CH₂-;
Z représente -CH₂-CH₃, -CH₂CH₂CH₃, -CH₂-CH₂-CH₂-CH₃, -CH(CH₃)-CH₃ ;
m est un entier variant de 0 à3 ;
n est un entier variant de 0 à 3 ;
o est un entier variant de 1 à 2 ; lesdits premier et second filtres sont présents dans les compositions selon l'invention dans une proportion produisant une activité synergique au niveau des facteurs de protection solaires conférés.

2. Composition selon la revendication1 où le ou les composés de formule (I) sont choisis parmi ceux de formule (la) suivante : 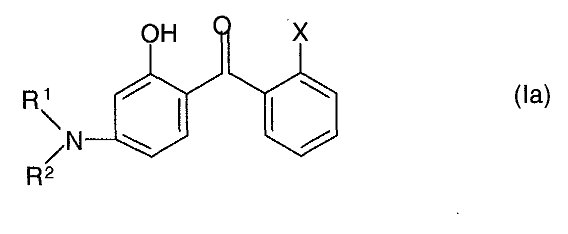 dans laquelle :
R¹ et R², identiques ou différents, désignent un atome d'hydrogène, un radical alkyle en C₁-C₁₂ ou forment avec l'atome d'azote avec lequel ils sont liés un cycle à 5 ou 6 chaînons ;
X désigne COOR⁵ ou CONR⁶R⁷;
R⁵ désigne un atome d'hydrogène, un radical alkyle en C₁-C₁₂, un radical cycloalkyle en C₃-C_{6.}
R⁶ et R⁷, identiques ou différents, désignent un atome d'hydrogène, un radical alkyle en C₁-C₁₂, un radical cycloalkyle en C₅-C₆.

3. Composition selon la revendication 2, où les composés de formule (la) sont ceux pour lesquels :
R¹ et R², identiques ou différents, désignent un radical alkyle en C₁-C₄ et plus particulèrement éthyle ;
R⁵ désigne un radical alkyle en C₃-C₈,
R⁶ et R⁷, identiques ou différents, désignent un radical alkyle en C₁-C₈,

4. Composition selon la revendication 1 où le ou les composés de formule (I) sont choisis parmi ceux de formule (Ib) suivante : 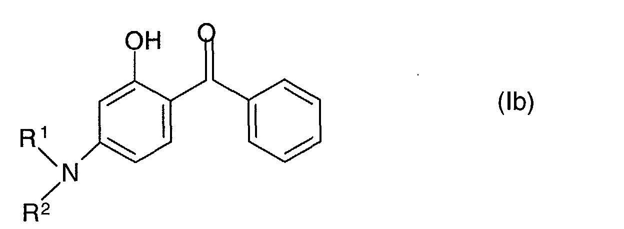 dans laquelle :
R¹ et R², identiques ou différents, désignent un radical alkyle en C₁-C₁₂ ou forment avec l'atome d'azote avec lequel ils sont liés un cycle à 5 ou 6 chaînons.

5. Composition selon la revendication 4, où le composé de formule (Ib) est choisi parmi:
- le (4-diéthylamino-2-hydroxyphényl)-phénylcétone.
- le (4-pyrrolidino-2-hydroxyphényl)-phénylcétone.

6. Composition selon la revendication 1 où le ou les composés de formule (I) sont choisis parmi ceux de formule (lc) suivante : 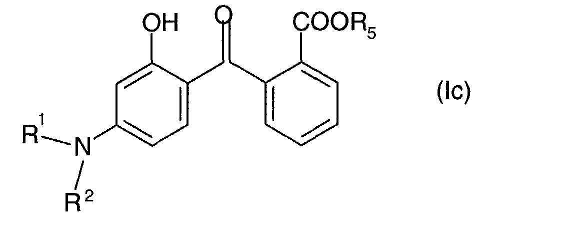 dans laquelle :
R¹ et R², identiques ou différents, désignent un atome d'hydrogène, un radical alkyle en C₁-C₈ ou forment avec l'atome d'azote avec lequel ils sont liés un cycle à 5 ou 6 chaînons ;
R⁵ désigne un atome d'hydrogène, un radical alkyle en C₁-C₁₂, un radical cycloalkyle en C₃-C₆.

7. Composition selon la revendication 6 où le composé de formule (lc) est choisi parmi:
- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

8. Composition selon la revendication 7 où le composé de formule (lc) est le 2-(4-diéthylamino-2-hydroxybenzoyl)-benzoate de n-hexyle

9. Composition selon l'une quelconque des revendications 1 à 8, dans laquelle le ou les dérivés de 2-hydroxybenzophénone aminosubstitués de formule (I) sont présents dans des proportions allant de 0,1 % à 15 % en poids

10. Composition selon la revendication 9, dans laquelle le ou les dérivés de 2-hydroxybenzophénone aminosubstitués de formule (I) sont présents dans des proportions allant de 1 à 10% en poids

11. Composition selon la revendication 10, dans laquelle le ou les dérivés de 2-hydroxybenzophénone aminosubstitués de formule (I) sont présents dans des proportions allant de 2 à 8% en poids par rapport au poids total de la composition.

12. Composition selon l'une quelconque des revendications 1 à 11, **caractérisée par le fait que** ledit premier filtre répond à la formule (II) suivante : 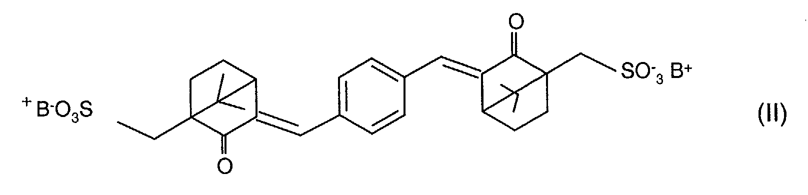 dans laquelle B désigne un atome d'hydrogène, un métal alcalin ou encore un radical NH(R)³+ dans lequel les radicaux R, qui peuvent être identiques ou différents, désignent un atome d'hydrogène, un radical alkyle ou hydroxyalkyle en C₁-C₄ ou encore un groupement Mn+/p, Mn+ désignant un cation métallique polyvalent dans lequel p est est égal à 2 ou 3 ou 4, Mn+ désignant de préférence un cation métallique choisi parmi Ca²+, Zn²+, Mg²+, Ba²+, Al³+ et Zr⁴+.

13. Composition selon la revendication 12, où l'acide benzène 1,4-di(3-méthylidène-10-camphosulfonique) est présent à des teneurs allant de 1 % à 10 % en poids, par rapport au poids total de la composition.

14. Composition selon la revendication 13, où l'acide benzène 1,4-di(3-méthylidène-10-camphosulfonique) est présent à des teneurs allant de 2 % à 8 % en poids, par rapport au poids total de la composition.

15. Composition selon l'une quelconque des revendications 1 à 14, **caractérisée par le fait qu'**elle contient en plus d'autres filtres organiques actifs dans l'UV-A et/ou l'UV-B.

16. Composition selon la revendication 15, où le ou les filtres UV organiques complémentaires sont choisis parmi les anthranilates ; les dérivés cinnamiques ; les dérivés salicyliques, les dérivés du camphre autres que le composé de formule (II); les dérivés de triazine ; les dérivés de la benzophénone autres que ceux de formule (I) ; les dérivés de β,β'-diphénylacrylate; les dérivés de benzotriazole ;les dérivés de benzalmalonate ; les dérivés de benzimidazole ; les imadazolines; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les 4,4-diarylbutadiènes.

17. Composition selon la revendication 16, **caractérisée par le fait que** le ou les filtres UV organiques sont choisis parmi les composés suivants :
- Ethylhexyl Salicylate,
- Octocrylene,
- Ethylhexyl Methoxycinnamate
- Butyl Methoxydibenzoylmethane,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Drometrizole Trisiloxane,
- Méthylène bis-Benzotriazolyl Tetramethylbutylphénol
- 1,1-dicarboxy (2,2'-diméthyl-propyl)-4,4-diphénylbutadiène.
et leurs mélanges.

18. Composition selon l'une quelconque des revendications 1 à 17, **caractérisée par le fait qu'**elle comprend en outre, des pigments ou des nanopigments d'oxydes métalliques, enrobés ou non.

19. Composition selon la revendication 18, **caractérisée par le fait que** lesdits pigments ou nanopigments sont choisis parmi les oxydes de titane, de zinc, de fer, de zirconium, de cérium et leurs mélanges, enrobés ou non.

20. Composition selon l'une quelconque des revendications 1 à 19, **caractérisée par le fait qu'**elle comprend en outre au moins un agent de bronzage et/ou de brunissage artificiel de la peau.

21. Composition selon l'une quelconque des revendications 1 à 20, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

22. Composition selon l'une quelconque des revendications 1 à 21, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

23. Composition selon l'une quelconque des revendications 1 à 22, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

24. Composition selon l'une quelconque des revendications 1 à 22, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

25. Utilisation d'une composition telle que définie dans les revendications 1 à 22 précédentes pour la fabrication de compositions cosmétiques ou dermatologiques destinées à la protection de la peau et/ou des cheveux contre le rayonnement ultraviolet, en particulier le rayonnement solaire.

26. Utilisation d'un dérivé de 2-hydroxybenzophénone aminosubstitué tel que défini dans les revendications 1 à 8 pour la fabrication de compositions cosmétiques ou dermatologiques destinées à la protection de la peau et/ou des cheveux contre le rayonnement ultraviolet, en particulier le rayonnement solaire comprenant au moins l'acide benzène 1,4-di-(3-méthylidène-10-camphosulfonique), éventuellement sous forme partiellement ou totalement neutralisée, dans le but de produire un effet synergique au niveau des indices de protection solaire conférés.
